(19) Europäisches Patentamt
European Patent Office
Office européen des brevets

(11) Veröffentlichungsnummer: **0 195 277**
**B1**

# (12) EUROPÄISCHE PATENTSCHRIFT

(45) Veröffentlichungstag der Patentschrift: **21.06.89**

(51) Int. Cl.⁴: **C 07 F 9/24**, C 07 F 9/141, C 07 F 9/201, C 07 F 9/65

(21) Anmeldenummer: **86102311.7**

(22) Anmeldetag: **22.02.86**

## (54) Verfahren und Mittel zur Phosphorylierung.

(30) Priorität: **06.03.85 DE 3507881**

(43) Veröffentlichungstag der Anmeldung: **24.09.86 Patentblatt 86/39**

(45) Bekanntmachung des Hinweises auf die Patenterteilung: **21.06.89 Patentblatt 89/25**

(84) Benannte Vertragsstaaten: **AT BE CH DE FR GB IT LI LU NL SE**

(56) Entgegenhaltungen:
**EP-A-0 131 993**
**DE-B-1 252 648**
**DE-C-1 075 584**

**CHEMICAL ABSTRACTS, Band 79, Nr. 9, 3. September 1973, Seite 385, Zusammenfassungsnr. 53549y, Columbus, Ohio, US; & PL - A - 66432 (MALINOWSKI et al.) 15.12.1972 in Verbindung mit CHEMICAL ABSTRACTS FORMELREGISTER, Band 79, Seite 245F, Spalte 3, Zeile 27**
**CHEMICAL ABSTRACTS, Band 101, Nr. 25, 17. Dezember 1984, Seite 809, Zusammenfassungsnr. 230935s, Columbus, Ohio, US; N.D. SINHA et al.: "Polymer support oligonucleotide synthesis XVIII: use of Beta-cyanoethyl-N,N-dialkylamino-/N-morpholino phosphoramidite of deoxynucleosides for the synthesis of DNA fragments simplifying deprotection and isolation of the final product", & NUCLEIC ACIDS RES. 984, 12(11), 4539-4557**
**CHEMICAL ABSTRACTS, Band 97, Nr. 21, 22. November 1982, Seite 840, Zusammenfassungsnr. 182538q, Columbus, Ohio, US; D.A. PREDVODITELEV et al.: "Acyl migration during phosphorylation of glycerides by phosphorus (III)**

(73) Patentinhaber: **HOECHST AKTIENGESELLSCHAFT, Postfach 80 03 20, D-6230 Frankfurt am Main 80 (DE)**

(72) Erfinder: **Engels, Joachim, Prof. Dr., Feldbergstrasse 1, D-6242 Kronberg/Taunus (DE)**
Erfinder: **Uhlmann, Eugen, Dr., Zum Talblick 31, D-6246 Glashütten/Taunus (DE)**

(56) Entgegenhaltungen: (Fortsetzung)
**acid amides" & ZH. ORG. KHIM. 1982, 18(6), 1326-1327**
**CHEMICAL ABSTRACTS, Band 95, Nr. 19, 9. November 1981, Seite 693, Zusammenfassungsnr. 169692n, Columbus, Ohio, US; E. NIFANT'EV et al.: "Synthesis of phosphatidylionols", & BIOORG.KHIM. 1981, 7(7), 1100-1106**
**CHEMICAL ABSTRACTS, Band 87, Nr. 23, 5. Dezember 1977, Seite 569, Zusammenfassungsnr. 183904m, Columbus, Ohio, US; J.G. LAMMERS et al.: "Synthesis of phospholipids via phospho triester intermediates", & RECL. TRAV. CHIM. PAYS-BAS 1977, 96(7-8), 216-218**
**CHEMICAL ABSTRACTS, Band 78, Nr. 9, 5. März, 1973, Seite 464, Zusammenfassungsnr. 57700t, Columbus, Ohio, US; E.E. NIFANT'EV et al.: "Acid amides of phosphorus acid as phosphorylating reagents", & ZH. OBSHCH. KHIM. 1972, 42(9), 1936-1939**
**TETRAHEDRON LETTERS, Band 23, Nr. 46, 1982, Seiten 4793-4796, GB; F. HIMMELSBACH et al.: "BIS-(p-Nitrophenylethyl) phosphoromonchloridate, a new versatile phosphorylating agent"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 195 277 B1

**Beschreibung**

Die Erfindung betrifft ein Verfahren zur Herstellung von Estern, Thioestern und Amiden der Phosphorsäure, Thiophosphorsäure und Selenophosphorsäure durch Umsetzung acylierbarer Hydroxy-, Mercapto- und Aminoverbindungen mit Phosphorigsäurediester-dialkylamiden, Oxidation des entstehenden Phosphorigsäuretriesters, -thioesters oder -diesteramids zur entsprechenden Verbindung mit fünfwertigem Phosphor und Abspaltung der beiden aus dem Phosphorylierungsreagenz stammenden alkoholischen bzw. phenolischen Gruppen mit Basen.

Die Erfindung betrifft insbesondere ein Verfahren zur Herstellung von Verbindungen der Formel I

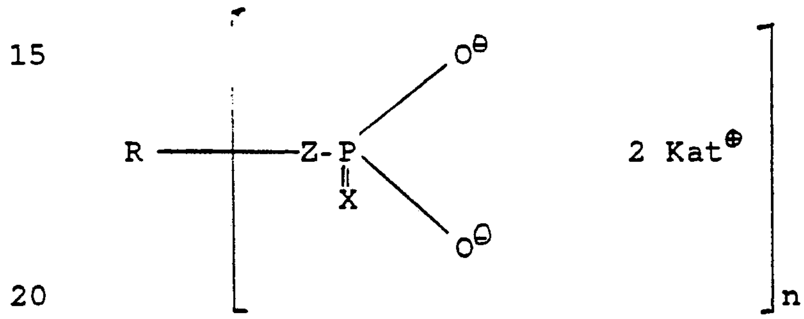

I

durch Phosphorylierung einer Verbindung der Formel II

$R\text{-}(\text{-}Z\text{-}H)_n$ II

mit mindestens n Mol eines Phosphorylierungsreagenzes, Oxidation der erhaltenen Verbindung und Freisetzung der Verbindung der Formel I mit Basen, dadurch gekennzeichnet, daß man ein Phosphorylierungsreagenz der Formel III

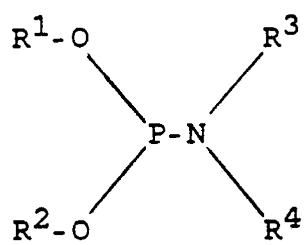

III

in Gegenwart einer schwachen Säure einsetzt, und die erhaltene Verbindung der Formel IV

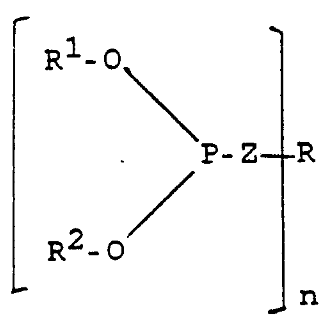

IV

zur Verbindung der Formel V oxidiert

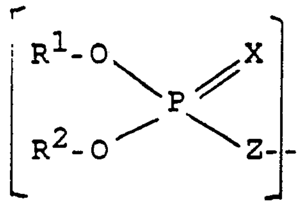

V

In den vorstehend genannten Formeln haben die Variablen die folgende Bedeutung:

R ist ein organischer Rest, der keine weiteren phosphorylierbaren Wasserstoffatome trägt,

X steht für Sauerstoff, Schwefel oder Selen

Z steht für Sauerstoff, Schwefel oder -NH-,

n bedeutet mindestens 1

Kat steht für ein Äquivalent eines Kations,

$R^1$ steht für eine Gruppe der Formel IIIa oder IIIb

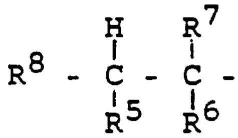

$R^9 - CH_2-$

IIIa IIIb

$R^5$, $R^6$und $R^7$, die gleich oder verschieden sein können, stehen für Wasserstoff oder Methyl,

$R^8$ steht für eine Gruppe der Formel IIIc, IIId, IIIe, Cyan oder Ar,

$-S-(O)_y-R_{10}$ $-Si(C_6H_5)_2-CH_3$ 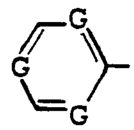

IIIc IIId IIIe

$R^{10}$ bedeutet Alkyl mit 1 bis 4 Kohlenstoffatomen oder Ar,

G, das gleich oder verschieden sein kann, steht für C oder N, wobei jedoch mindestens ein G für N steht,

Ar steht für Phenyl, das durch 1 bis 5 gleiche oder verschiedene Substituenten der Reihe Chlor, Fluor, Nitro und Cyan oder zusätzlich zu den elektronenabziehenden Substituenten durch einen oder zwei lipophile Reste substituiert sein kann,

y steht für 0, 1 oder 2,

$R^9$ steht für eine Gruppe der Formel IIIf oder IIIg,

$CY_3$ 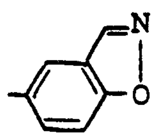

IIIf IIIg

Y steht für gleiche oder verschiedene Substituenten der Reihe Chlor, Brom oder Jod,

$R^2$ kann eine Bedeutung von $R^1$ haben und dann gleich oder verschieden sein, oder ein Alkyl mit 1 bis 4 Kohlenstoffatomen, eine Gruppe der Formel IIIh

$-CH_2-Ar$ IIIh

oder auch Ar bedeuten, wenn dieses alkalisch leichter abspaltbar ist als $R^1$,

$R^5$ und $R^8$ können zusammen für eine Gruppe der Formel IIIi

IIIi

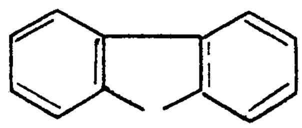

IIIi

stehen und

$R^1$ kann auch für Methyl stehen, wenn $R^2$ ebenfalls Methyl ist.

Die Natur der Gruppen $R^3$ und $R^4$ ist unkritisch, sofern der Rest $-NR^3R^4$ bei der Umsetzung mit der Verbindung der Formel II abgespalten wird.

$R^3$ und $R^4$, die gleich oder verschieden sein können, stehen für Alkyl mit 1 bis 8 Kohlenstoffatomen, Cycloalkyl mit 5 bis 12, vorzugsweise bis 8 Kohlenstoffatomen, Benzyl oder Phenyl oder zusammen mit dem Stickstoffatom, an das sie gebunden sind, für einen gesättigten oder ungesättigten heterocyclischen Ring, der gegebenenfalls weitere Heteroatome und Substituenten enthalten kann.

Bevorzugte Bedeutungen der vorstehend genannten Variablen sind:

$R^5$, $R^6$ und $R^7$ bedeuten Wasserstoff,

$R^8$ bedeutet Cyan, Phenyl oder nitriertes Phenyl, insbesondere Mononitrophenyl wie p-Nitrophenyl oder Dinitrophenyl wie 2,5-Dinitrophenyl, chloriertes Phenyl, insbesondere Mono-, Di- oder Trichlorphenyl oder 2-Chlor-4-nitrophenyl sowie Nitrophenyl, das durch einen oder zwei Kohlenwasserstoffreste, insbesondere Alkanreste, mit 8 bis 24, vor allem 12 bis 18, Kohlenstoffatomen substituiert ist.

$R^9$ steht bevorzugt für eine Gruppe der Formel IIIf, wobei Y bevorzugt für Chlor steht.

$R^2$ ist niederes Alkyl, insbesondere Methyl, oder es hat die Bedeutung von $R^1$ und ist dann vorteilhaft identisch mit $R^1$.

$R^3$ und $R^4$ stehen für Alkyl mit 1 bis 6 Kohlenstoffatomen, insbesondere für niederes Alkyl, Cyclohexyl, Benzyl oder Phenyl und zusammen mit dem Stickstoffatom für Pyrrolidyl, Piperidyl, Morpholyl, Triazolo, Benzotriazolo oder Tetrazolo, wobei die aromatischen Reste durch Chlor, Fluor, Nitro oder Cyan substituiert sein können.

Der Ausdruck "nieder" steht für Reste mit bis zu 4 Kohlenstoffatomen.

X und Z stehen bevorzugt für Sauerstoff.

n steht für 1 bis 6, vor allem 1 oder 2, insbesondere 1.

Die Verbindungen der Formel III, in denen $R^1$ von Methyl verschieden ist, sind neu, ebenso die Verbindungen der Formel VI. Diese neuen Verbindungen sind ebenfalls Gegenstand der Erfindung.

Aus F. Himmelsbach et al., Tetrahedron Letters 23 (1982) 4793 - 4796 sind Verbindungen der Formel V bekannt, in denen $R^1$ und $R^2$ für 2-(4-Nitrophenyl)ethyl, X und Z für Sauerstoff und R für einen Thymidylrest steht. Diese Verbindungen werden durch Umsetzung von Thymidin mit Bis-(p-nitrophenylethyl)-phosphoromonochloridat erhalten, wobei im wesentlichen die 5'-ständige Hydroxygruppe acyliert wird. Dieses Phosphorylierungsreagenz ist ziemlich labil und konnte weder unter Hochvakuum destilliert noch in fester Form hergestellt werden. Wenn man vom Phosphortrichlorid ausgeht, so erfordert die Herstellung von Verbindungen der Formel V auf dem bekannten Wege fünf Reaktionsstufen, während diese Verbindungen auf dem erfindungsgemäßen Wege in vier Schritten herstellbar sind.

Nach der genannten Literaturstelle werden die Verbindungen der Formel V dann weiter zu Oligonucleotiden umgesetzt und aus diesen schließlich die p-Nitrophenylethylgruppen mit 1,5-Diazabicyclo[5,4,0]undecen-5 (DBU) abgespalten.

In der Druckschrift EP-A-0 131 993 werden Phosphorylierungsreagenzien mit mindestens einer geschützten Hydroxylgruppe und mit mindestens einer Abgangsgruppe offenbart, die dadurch gekennzeichnet sind, daß als Hydroxylschutzgruppe Methylsulfonylethyl (Mse) verwendet wird. Es wird als Abgangsgruppe auch Triazolyl und Tetrazolyl beschrieben, wobei jedoch offenbleibt, ob eine P-C oder P-N-Bindung vorliegt.

In dem erfindungsgemäßen Verfahren, das dadurch gekennzeichnet ist, daß Phosphorigsäureesteramide in Gegenwart einer schwachen Säure eingesetzt werden, ist überraschenderweise die Spezifität der Phosphorylierungsreaktion erhöht, da die Gefahr einer unspezifischen Abspaltung der Schutzgruppen - die generell basisch abgespalten werden - verringert wird. Eine Abspaltung der Schutzgruppen unter Phosphorylierungsbedingungen führt zu ungeschützten phosphorylierbaren Gruppen, die im Verlauf der Reaktion mit einem weiteren Molekül Phosphorylierungsreagenz reagieren können. Ferner können im beanspruchten Verfahren Schutzgruppen eingesetzt werden, die unter den in der EP-A-0 133 993 beschriebenen Reaktionsbedingungen (neutral bis schwach alkalisch) nur wenig oder gar nicht stabil sind.

Die Verbindungen der Formel III sind nach an sich bekannten Methoden aus Phosphortrichlorid durch Umsetzung mit den Hydroxyverbindungen $R^1$-OH und $R^2$-OH und dem sekundären Amin $H-NR^3R^4$ zugänglich. Anstelle des freien sekundären Amins kann auch eine entsprechende Silylverbindung eingesetzt werden. Die Reihenfolge des Austausches der Chloratome im Phosphortrichlorid richtet sich nach der Reaktivität der Hydroxy- bzw. Aminoverbindungen. So kann man beispielsweise die Verbindung der Formel VI

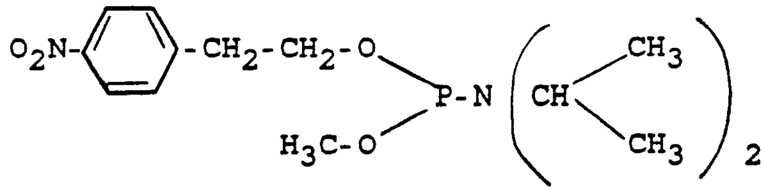

VI

auf folgenden Wegen gewinnen:

a) Man setzt zunächst Phosphortrichlorid mit Methanol in Gegenwart einer Base um, setzt das Reaktionsprodukt mit Diisopropylamin und anschließend mit dem 2-(p-Nitrophenyl)-ethanol in Gegenwart einer Base um,

b) man setzt das Phosphortrichlorid zunächst mit dem 2-(p-Nitrophenyl)-ethanol, dann mit dem 1sopropylamin und schließlich mit Methanol um oder

c) man läßt das Phosphortrichlorid zuerst mit dem Diisopropylamin reagieren und setzt dann mit dem 2-(p-Nitrophenyl)-ethanol und hierauf mit Methanol um.

Die Umsetzung des Phosphorylierungsreagenzes der allgemeinen Formel III mit einer Hydroxyverbindung erfolgt entweder in Lösung mit einer äquimolaren Menge bzw. einem geringen Überschuß von beispielsweise bis zu 50, vorzugsweise bis zu 25, Mol-% an Phosphorylierungsreagenz in Gegenwart einer schwachen Säure wie Tetrazol oder einem Aminhydrochlorid oder in Form einer Festphasen-Synthese mit einem großen Überschuß an Phosphorylierungsreagenz in Gegenwart einer schwachen Säure. Wenn beispielsweise die Hydroxykomponente trägergebunden ist, wird man das Phosphorylierungsreagenz in einem 10- bis 20-fachen molaren Überschuß einsetzen. Wenn man in Lösung arbeitet, muß das Lösemittel selbstverständlich gegenüber dem Phosphorylierungsreagenz und der zu acylierenden Komponente inert sein. Die Umsetzung erfolgt bei Temperaturen von -80 bis +80° C, vorzugsweise -20 bis +50° C, insbesondere 0 bis 25° C.

Das Phosphorylierungsprodukt der Formel IV hat je nach Wahl Reste $R^1$ und $R^2$ einen mehr oder weniger ausgeprägten lipophilen Charakter, insbesondere dann, wenn $R^8$ ein lipophil substituiertes Phenyl darstellt. Diese lipophilen Verbindungen lassen sich einfach von den nicht phosphorylierten Ausgangsmaterialien abtrennen, beispielsweise durch Chromatographie wie Hochdruck-Flüssigkeitschromatographie auf "reversed phase"-Material, beispielsweise das handelsübliche Silicagel mit Octadecylsilylgruppen.

Die Wahl der Substituenten $R^1$ und $R^2$ richtet sich selbstverständlich nach der chemischen Natur des Restes R, also nach derjenigen Verbindung, in die die Phosphatgruppe eingeführt werden soll. Unter der Definition von R als einer Gruppe, die keine weiteren phosphorylierbaren H-Z-Gruppen enthält, soll verstanden werden, daß das Phosphorylierungsreagenz der Formel III relativ unspezifisch acyliert und somit bei einer gezielten Phosphorylierung in allen weiteren gegebenenfallls vorhandenen phosphorylierbaren Z-H-Funktionen die Wasserstoffatome durch entsprechende Schutzgruppen ersetzt sind. Unter "phosphorylierbar" sollen hier solche Z-H-Funktionen verstanden werden, die unter den Acylierungsbedingungen zu einer Umsetzung befähigt sind - durch sterische Hinderung oder inaktivierende Gruppen in ihrem Reaktionsvermögen beeinträchtigte Z-H-Funktionen sollen also ausgeschlossen sein.

Da die Reste $R^1$ und $R^2$ zur Freisetzung der Phosphatgruppe in den meisten Fällen mit Hilfe einer Base abgespalten werden, richtet sich die Natur der Reste $R^1$ und $R^2$ und der eingesetzten Base nach der Empfindlichkeit des Restes R. Bei entsprechender Stabilität von R kann also die Abspaltung mit wäßrigen Alkalilaugen erfolgen, womit auch die Wahl von $R^1$ und $R^2$ relativ unkritisch ist. Bei empfindlichen Gruppen am Rest R wird man in an sich bekannter Weise unter entsprechend schonenden Bedingungen arbeiten, beispielsweise im nicht-wäßrigen System und unter Verwendung einer geeigneten Base, beispielsweise dem aus der vorstehend diskutierten Literaturstelle bekannten DBU.

Die Abspaltung der Reste $R^1$ und $R^2$ erfolgt nach an sich bekannten Methoden:

Die Abspaltung eines Methylrestes aus einem Phosphattriester kann mit Thiophenol oder Thiokresol in Gegenwart einer Base wie Triethylamin erfolgen. Diese Reaktion erfolgt zweckmäßig bei Temperaturen von -100 bis +100°C, vorzugsweise bei -60 bis +60°C, insbesondere bei -20 bis +30°C. Die in einer ß-Eliminierungsreaktion entfernbaren Gruppen, also beispielsweise Verbindungen, in denen die Gruppe IIIb enthalten ist, entfernt man mit Metallen wie Zink oder durch potentialgesteuerte Elektroreduktion. Diese Reaktion erfolgt zweckmäßig bei Temperaturen von -50 bis +80°C, vorzugsweise bei -20 bis -50°C, insbesondere bei 0 bis 30° C. Elektronegativ substituierte Ethylgruppen lassen sich mit nichtnucleophilen Basen entfernen, die auch an polymere Träger gebunden sein können. Diese Reaktion erfolgt zweckmäßig bei Temperaturen von -60 bis +100° C, vorzugsweise bei -20 bis +50° C, insbesondere 0 bis 30° C.

Bei der Abspaltung von elektronegativ substituierten Ethylgruppen aus Verbindungen der Formel V werden vorteilhaft die folgenden Basen eingesetzt, die nach abnehmender Reaktivität aufgeführt sind:

a) cyclische Phosphorsäuretriamid-imide der Formel VII

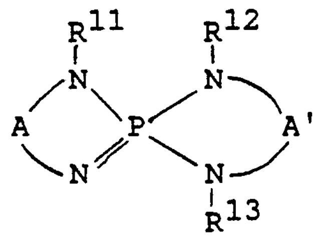

VII

in der $R^{11}$, $R^{12}$ und $R^{13}$ gleiche oder verschiedene niedere Alkylgruppen, vorzugsweise Methyl oder Ethyl, und A und A' niedere Alkylengruppen mit 2 bis 4 Kohlenstoffatomen in der Kette, vorzugsweise 1,3-Propylen, bedeuten,

b) 1,5,7-Triazabicyclo[4,4,0]dec-5-en (TBD) sowie 7-Methyl 1,5,7-triazabicyclo[4,4,0]dec-5-en,

c) 1,5-Diazabicyclo[4,3,0]non-5-en (DBN), DBU sowie niedere Alkalimetallalkoholate, insbesondere Natrium- und Kaliumalkanolate wie Natriummethylat, Natriumethylat oder Kaliumtertiärbutylat und

d) 1,1,3,3-Tetramethylguanidin.

Verbindungen, in denen $R^1$ und gegebenenfalls auch $R^2$ ß-Cyanethyl sind, lassen sich mit Ammoniak spalten und werden deshalb bevorzugt dort eingesetzt, wo andere Schutzgruppen mit Hilfe von Ammoniak abgespalten werden.

Ein großer Vorteil des erfindungsgemäßen Verfahrens liegt darin, daß empfindliche Verbindungen phosphoryliert werden können. Wie vorstehend bei der Abspaltung der Reste $R^1$ und $R^2$ mit Basen gezeigt wurde, erlaubt das erfindungsgemäße Verfahren eine Vielfalt von Ausgestaltungen, so daß die einzelnen Reaktionsschritte mit weiteren Reaktionen am Rest R koordiniert werden können. Dieser Gesichtspunkt gilt nicht nur für die Abspaltung der Reste $R^1$ und $R^2$, sondern genauso für die Oxidationsreaktion und - wenn gewünscht - für das Einführen von mehr als einer Phosphatgruppe.

Ein weiterer erheblicher Vorteil der Erfindung ist darin zu sehen, daß nicht nur die Phosphatgruppe selbst, sondern auch die Thio- und Seleno-Phosphatgruppe eingeführt werden kann. Man kommt auf diesem Wege einfach zu chemischen Analogen von Naturstoffen, die beispielsweise nach den Verfahren von Himmelsbach et al. nicht zugänglich sind.

Die Oxidation der Verbindungen der allgemeinen Formel IV zu den Phosphaten der allgemeinen Formel V (X = O) erfolgt in der für analoge Verbindungen bekannten Weise. Als Oxidationsmittel können beispielsweise Distickstofftetroxid oder Jod, insbesondere aber Peroxide, vor allem wasserfreies t-Butylhydroperoxid, eingesetzt werden. Die Reaktion wird bevorzugt in einem mäßig polaren Lösemittel durchgeführt, beispielsweise in Nitrilen wie Acetonitril oder halogenierten, insbesondere chlorierten niederen Kohlenwasserstoffen wie Chloroform. Diese Reaktion erfolgt zweckmäßig bei Temperaturen von -100 bis +100° C, vorzugsweise bei -50 bis +60° C, insbesondere bei -20 bis +30° C.

Die Herstellung der Verbindungen der allgemeinen Formel V, in der X Schwefel oder Selen bedeutet, erfolgt durch direkte Umsetzung der Verbindungen der allgemeinen Formel IV mit elementarem Schwefel oder Selen. Rühren in einem polaren Lösemittel wie Tetrahydrofuran mit der stöchiometrischen Menge an Schwefel oder Selen führt in guten Ausbeuten zu den entsprechenden Thio- oder Selenophosphaten der allgemeinen Formel V. Diese Reaktion erfolgt zweckmäßig bei Temperaturen von -100 bis +100° C, vorzugsweise bei -50 bis +60° C, insbesondere bei -20 bis +30° C.

Es wurde bereits betont, daß es die Erfindung erlaubt, auf die chemische Natur des Restes R in erheblichem Ausmaß Rücksicht zu nehmen und daß dieser Rest auch empfindliche Gruppen oder Molekülteile tragen kann. Als Ausgangsmaterialien der Formel II kommen somit aliphatische wie aromatische Hydroxy-, Mercapto- und Aminoverbindungen in Betracht, also Alkohole, Phenole, Mercaptane, Thiophenole und aliphatische und aromatische Amine. Besonders vorteilhaft können Naturstoffe oder Naturstoffanaloge Verbindungen phosphoryliert werden, beispielsweise Steroidderivate, Phospholipide und vor allem Zucker und Zuckerderivate wie Nucleoside, Nucleotide, Oligonucleotide oder Polynucleotide.

Die Darstellung von Nucleotiden mit endständiger ungeschützter Phosphatfunktion ist mit Hilfe der derzeit verwendeten Phosphatschutzgruppen-Kombination nach dem Phosphit-Verfahren nicht oder nur mit einem enormen zusätzlichen Aufwand möglich. Auch die bei der Triester-Synthese häufig verwendete Schutzgruppenkombination des o-Chlorphenyl-ß-cyanoethylphosphats liefert keine endständigen Phosphate, da die Hydrolyse der o-Chlorphenyl-Gruppe unter Erhaltung der ß-Cyanoethyl-Gruppe nicht möglich ist.

Nach den bisher bekannten Methoden wurde deshalb die Phosphorylierung der endständigen 5'-Hydroxygruppe mit Hilfe eines Nucleosidtriphosphats, üblicherweise Adenosintriphosphat, und einer Kinase wie (T4)-Polynucleotidkinase vorgenommen. Dieses Verfahren ist recht aufwendig.

Erfindungsgemäß kann nun die Phosphorylierung der 5'-Hydroxygruppe auf chemischem Weg erfolgen, und zwar nicht nur am isolierten Oligonucleotid, sondern sogar schon an der noch trägergebundenden Verbindung. Das erfindungsgemäße Verfahren läuft unter so schonenden Bedingungen, daß das Nucleosid, Nucleotid, Oligo- oder Polynucleotid nicht geschädigt wird.

Die Erfindung erlaubt aber auch die Einführung von Phosphatgruppen an anderer Stelle in Nucleoside oder monomere, oligomere oder polymere Nucleotide, beispielsweise durch Phosphorylierung der 3'-Hydroxygruppe oder -gruppen oder bei Ribonucleosiden oder Ribonucleotiden eine Einführung in die 2'-Position.

Die Erfindung wird in den folgenden Beispielen auf dem bekanntermaßen schwierigen Gebiet der Nucleotide näher erläutert. Hierin ist jedoch keinesfalls eine Beschränkung zu sehen, da die Phosphorylierung von Nucleotiden ganz allgemein repräsentativ fur die Einführung von Phosphatgruppen ist.

Herstellung der Phosphorylierungsreagenzien der allgemeinen Formel III

**Beispiel 1**

Phosphorigsäure-methylester-diisopropylamid-chlorid

Man legt 127 g (0,96 mol) Phosphorigsäuremechylester-dichlorid in einer Mischung aus 250 ml absolutem Ether und 250 ml absolutem Pentan vor und tropft unter starkem Rühren und Stickstoffschutz bei -15°C eine Lösung von 270 ml (1,92 mol) Diisopropylamin in 100 ml absolutem Ether und 100 ml absolutem Pentan im Lauf von 2 Stunden zu. Man läßt das Reaktionsgemisch unter Rühren sich auf Raumtemperatur erwärmen und rührt noch 2 Stunden weiter. Dann kühlt man wieder auf -15°C ab und trennt das Hydrochlorid durch Filtration unter Inertgas von der Reaktionslösung ab. Der Filterkuchen wird mit 750 ml eines kalten Gemischs aus gleichen Volumina aus absolutem Ether und absolutem Pentan nachgewaschen. Die Filtrate werden vereinigt und die Lösungsmittel im Wasserstrahlvakuum abgezogen. Der Rückstand wird zweimal einer Hochvakuumdestillation über eine 80 cm Vigreux-Kolonne unterworfen. Man erhält 80 g einer Hauptfraktion (Siedepunkt 38 bis 41°C bei 0,1 mbar), die gemäß $^{31}$P-NMR zu 99 % einheitlich ist ($\delta$ = 185 ppm).

**Beispiel 2**

Phosphorigsäure-methylester-[(p-nitrophenyl)-ethylester]-diisopropylamid (Formel VI)

5,01 g (30 mmol) scharf getrocknetes 2-(p-Nitrophenyl)-ethanol werden in 60 ml frisch destilliertem Methylenchlorid gelöst und mit 24 ml Diisopropyl-ethyl-amin versetzt. Man kühlt auf etwa 0°C und tropft im Lauf von 5 Minuten unter Rühren 6 ml (33 mmol) Phosphorigsäuremethylester-diisopropylamid-chlorid zu und rührt noch 10 Minuten bei dieser Temperatur weiter. Dann verdünnt man das Reaktionsgemisch mit 750 ml Essigester, der zuvor mit 450 ml Phosphatpuffer vom pH 7 gewaschen wurde. Die Essigesterphase wird dreimal mit 450 ml Phosphatpuffer (pH 7) ausgeschüttelt, über Natriumsulfat getrocknet und eingeengt. Das anfallende Öl wird im Hochvakuum bei 30°C getrocknet. Man erhält 9 g Öl, das nach $^{31}$P-NMR 86-%-ig ist. Durch Chromatographie über eine kurze Kieselgelsäule, die mit Essigester/Methylenchlorid/Triethylamin im Volumenverhältnis 5 : 4 : 1 äquilibrieret wurde, kann das Produkt mit dem genannten Eluentensystem weiter angereichert werden ($^{31}$P-NMR: 95-%-ig). Man erhält ein Öl, das im Kühlschrank (-20°C) erstarrt.

| | | |
|---|---|---|
| $^{31}$P-NMR: | $\delta$ = 149 ppm | |
| $^{1}$H-NMR: | P-O-$\underline{CH_2}$-$CH_2$- | $\delta$ = 3,75 ppm, m [2] |
| | P-O-$CH_2$-$\underline{CH_2}$- | $\delta$ = 2,98 ppm, t [2] |
| | P-$\underline{OCH_3}$ | $\delta$ = 3,35 ppm, d [3] |

m = Multiplett [Intensität]
s = Singulett
d = Dublett
t = Triplett

**Beispiel 3**

Phosphorigsäure-methylester-2-cyanoethylester-diisopropylamid (Formel VIII)

30 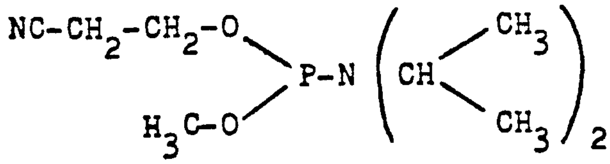 VIII

Analog Beispiel 2 setzt man 0,68 ml (10 mmol) 2-Cyanoethanol mit 2 ml (11 mmol) Phosphorigsäure-methylester-diisopropylamid-chlorid in Gegenwart von 8 ml Diisopropylethyl-amin in 20 ml frisch destilliertem Methylenchlorid um. Nach Trocknen bei 30°C im Hochvakuum erhält man 2,5 g eines Öls, das laut $^{31}$P-NMR 94 % der Verbindung VIII enthält.

31P-NMR: δ = 150 ppm
$^{1}$H-NMR: P-$\underline{OCH_2}CH_2$-CN δ = 3,75 ppm, m [2]
P-$OCH_2\underline{CH_2}$-CN δ = 2,60 ppm, t [2]
P-$\underline{OCH_3}$ δ = 3,35 ppm, d [3]

**Beispeiel 4**

Phosphorigsäure-2-(p-nitrophenyl)-ethylester-dichlorid

50 g (30 mmol) 2-(p-Nitrophenyl)-ethanol werden in 100 ml absolutem Ether gelöst und im Lauf von 40 Minuten bei -20 bis -30°C unter Rühren tropfenweise mit 25,5 ml (150 mmol) frisch destilliertem Posphortrichlorid umgesetzt. Man zieht bei 0°C den Ether im Wasserstrahlvakuum und anschließend das überschüssige Phosphortrichlorid im Hochvakuum ab und trocknet das zurückbleibende Öl im Hochvakuum.

$^{31}$P-NMR: δ = 179,8 ppm 93 % des Gesamt-P.

**Beispiel 5**

Phosphorigsäure-diisopropylamid-dichlorid

87 ml (1 mol) Phosphortrichlorid werden in 800 ml Petrolether gelöst, die Lösung auf 5°C gekühlt und dann eine Lösung von 283 ml (2 mol) Diisopropylamin in 660 ml Petrolether im Lauf einer Stunde bei 5°C zugetropft. Man rührt eine Stunde bei dieser Temperatur nach, filtriert das abgeschiedene Aminhydrochlorid ab, zieht den Petrolether unter Wasserstrahlvakuum ab und destilliert den Rückstand im Vakuum (0,2 mbar). Man erhält als Hauptfraktion bei 38 bis 41°C 98 g eines Öls, das beim Stehen im Kühlschrank (-20°C) kristallisiert und zu 99 % rein ist ($^{31}$P-NMR, δ = 170 ppm).

**Beispiel 6**

Phosphorigsäure-bis-[2-(p-nitrophenyl)-ethylester]-diisopropylamid (Formel IX)

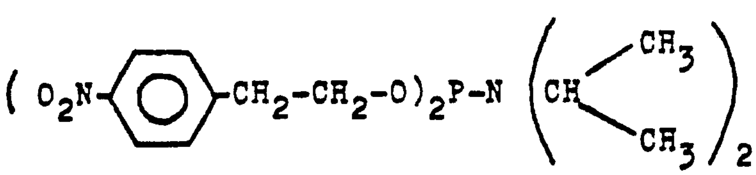 IX

Eine Lösung von 16 (0,1 mol) 2-(p-Nitrophenyl)-ethanol in 40 ml absolutem Tetrahydrofuran wird mit 26 ml (0,15 mol) Diisopropylethylamin versetzt und auf 0°C gekühlt. Unter Rühren tropft man innerhalb von 15 Minuten 10 g

(0,05 mol) Phosphorigsäure-diisopropylamid-dichlorid zu. Dann läßt man das Reaktionsgemisch auf Raumtemperatur kommen und noch etwa 30 Minuten weiterreagieren. Man saugt von Aminhydrochlorid ab, wäscht den Filterkuchen mit wenig Tetrahydrofuran und engt die vereinigten Filtrate ein, wobei man einen festen Rückstand erhält. 1 g dieses Rückstandes wird durch Säulenchromatographie an 70 g Kieselgel mit Toluol/n-Hexan/Triethylamin im Volumenverhältnis 7 : 2 : 1 gereinigt. Man erhält 0,95 g eines Pulvers, das laut $^{31}$P-NMR zu 96 % rein ist.

$^{31}$P-NMR: $\delta = 147,5$ ppm
$^{1}$H-NMR: P-$\underline{OCH_2}CH_2$- $\delta = 3,85$ ppm, m [4]
P-$OCH_2\underline{CH_2}$- $\delta = 2,98$ ppm, t [4]
P-N-$\underline{CH}$ $\delta = 3,5$ ppm, m [2]
P-N-$CH(\underline{CH_3})_2$ $\delta = 1,13$ ppm, d [12]

Phosphorylierungsreaktionen:

Chemische Synthese eines einzelsträngigen Oligonucleotids mit einem 5'-Phosphat-Rest: NcoI-Linker 5' pCCATGG 3'.

**Beispiel 7**

Nach der Methode von M. J. Gait et al., Nucleic Acids Res. 8 (1980), 1081 - 1096, wird das am 3'-Ende stehende Nucleosid, im vorliegenden Falle also Guanosin, an "controlled pore glass" (CPG, Firma Pierce) über die 3'-Hydroxyfunktion kovalent gebunden.

Hierzu wird zunächst das Kieselgel unter Abspaltung von Ethanol mit 3-(Triethoxysilyl)-propylamin umgesetzt, wobei eine Si-O-Si-Bindung entsteht. Das Guanosin wird als $N^{2'}$- Isobutyryl-3'-O-succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylamingruppe acyliert.

In den folgenden Syntheseschritten wird die Basenkomponente als 5'-O-Dimethoxytrityl-nucleosid-3'-phosphorigsäure-monomethylester-dialkylamid oder -chlorid eingesetzt, wobei das Adenin als $N^6$-Benzoyl-Verbindung, das Cytosin als $N^4$-Benzoyl-Verbindung, das Guanin als $N^2$-Isobutyryl-Verbindung und das keine Aminogruppe enthaltende Thymin ohne Schutzgruppe vorliegen.

Reaktionszyklus für den Aufbau der Oligonucleotidkette: 25 mg des polymeren Trägers, der 1 µmol Guanosin gebunden enthält, werden nacheinander mit folgenden Agentien behandelt:

a) Nitromethan
b) 3 % Trichloressigsäure in 1,2-Dichlorethan
c) Nitromethan
d) Acetonitril
e) 10 bis 20 µmol des entsprechenden Nucleosidphosphits und 100 µmol Tetrazol in 0,2 ml wasserfreiem Acetonitril (5 min)
f) 20 % Acetanhydrid in Tetrahydrofuran mit 40 % Lutidin und 10 % Dimethylaminopyridin (2 min)
g) Acetonitril
h) Tetrahydrofuran mit 20 % Wasser und 40 % Lutidin
i) 3 % Jod in Collidin/Wasser/Tetrahydrofuran im Volumenverhältnis 5 : 4 : 1 (0,5 min)
j) Acetonitril

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion (b) spektrophotometrisch durch Messung der Absorption des Dimethoxytritylkations bei einer Wellenlänge von 496 nm bestimmt werden.

Der Reaktionszyklus für die chemische Phosphorylierung der 5'-Hydroxyfunktion erfolgt im Prinzip wie in den vorherigen Beispielen, jedoch mit dem Unterschied, daß einerseits bei e) 20 µmol des entsprechenden Phosphorylierungsreagenzes (z. B. die Verbindung VI) und 100 µmol Tetrazol in 0,2 ml wasserfreiem Tetrahydrofuran/Acetonitril (Mischung aus gleichen Volumina) 10 bis 20 Minuten zur Reaktion gebracht werden, andererseits der Schritt f) wegfällt und drittens die Zeit im Schritt i) auf 5 Minuten verlängert wird.

Nach abgeschlossener Synthese des Oligonucleotids werden die Methylphosphatschutzgruppen des Oligomers mit Hilfe von p-Thiokresol und Triethylamin abgespalten. Anschliessend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab.

a) Im Falle des Phosphorylierungsreagenzes Phosphorigsäure-methylester-[2-(p-nitrophenyl)-ethylester]-diisopropylamid (VI) erhält man nach dieser Schutzgruppenabspaltung ein Oligonucleotid mit einem 2-(p-Nitrophenyl)-ethylphosphatrest an der 5'-Hydroxygruppe. Dieser Phosphatester kann durch HPLC auf

"reversed phase"-Material gereinigt werden, was zu einer besonders guten Abtrennung von der Hydroxykomponente führt. Zur Abspaltung der p-Nitrophenylethyl-Gruppe behandelt man den Ester mit 0,9 M TBD in Pyridin/Wasser im Volumenverhältnis 9 : 1 8 Stunden bei Raumtemperatur. Danach ist laut HPLC bzw. Gelelektrophorese auf einem 20-%-igen Polyacrylamidgel mit 7 M Harnstoff kein Ausgangsmaterial mehr vorhanden. Das Reaktionsgemisch wird mit 1 ml 2 M Essigsäure versetzt und eingeengt. Den Rückstand entsalzt man über eine Säule mit ®SEPHADEX G 50 mit 0,02 M Triethylammoniumbicarbonat (pH 7) als Eluent.

Das resultierende 5'-Phosphat-Oligonucleotid kann mit Hilfe von Polynucleotid-Ligase zu doppelsträngiger repetitiver DNA polymerisiert werden, die sich dann mit dem Restriktionsenzym Hae III (Erkennung: GG ↓ CC) wieder schneiden läßt.

b) Im Falle des Phosphorylierungsreagenzes Phosphorigsäure-metyhlester-2-cyanoethylester-diisopropylamid (VIII) und des Phosphorigsäure-bis-(2-cyanoethylester)-diisopropylamid (X)

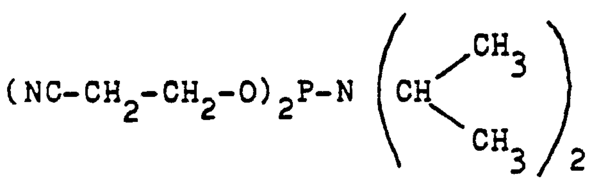

X

erhält man nach der Ammoniakspaltung (konzentriertes Ammoniak, 3 Tage Raumtemperatur oder 10 Stunden bei 75° C) gleich das freie 5'-Phosphat p CCATGG.

c) Im Falle des Reagenzes Phosphorigsäure-bis-[2-(p-nitrophenyl)-ethylester]-diisopropylamid (IX) erhält man nach der Thiophenolat- und Ammoniakbehandlung auch den Phosphat-2-(p-nitrophenyl)-ethylester, der wiederum in das 5'-Phosphat schutzgruppenfrei mit einer geeigneten Base wie DBU oder TBD gespalten werden kann.

**Patentansprüche** für die Vertragsstaaten: BE, CH, DE, FR, GB, IT, LI, LU, NL, SE

1. Verfahren zur Herstellung von Verbindungen der Formel I

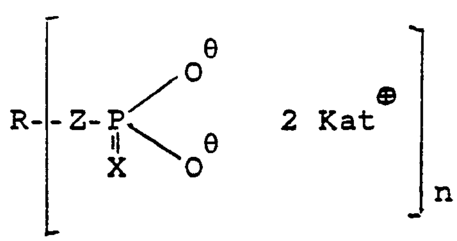

I

in der

Kat für ein Äquivalent eines Kations steht,
R ein organischer Rest ist, der keine weiteren phosphorylierbaren Wasserstoffatome trägt,
X für Sauerstoff, Schwefel oder Selen steht,
Z für Sauerstoff, Schwefel oder -NH- steht, und
n mindestens 1 bedeutet,

durch Phosphorylierung einer Verbindung der Formel II

$R\text{-}(\text{-}Z\text{-}H)_n$ II

mit mindestens n Mol eines Phosphorylierungsreagenzes, Oxidation der erhaltenen Verbindung und Freisetzung der Verbindung der Formel I mit Basen, dadurch gekennzeichnet, daß man in Gegenwart einer schwachen Säure ein Phosphorylierungsreagens der Formel III

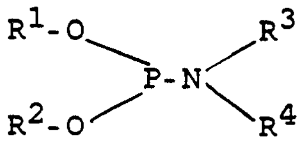

III

einsetzt, in der $R^1$ für eine Gruppe der Formel IIIa oder IIIb steht,

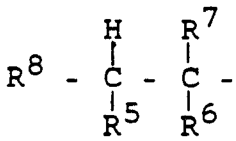

$R^9 - CH_2 -$

IIIa IIIb

$R^5$, $R^6$ und $R^7$, die gleich oder verschieden sein können, Wasserstoff oder Methyl bedeuten,
$R^8$ eine Gruppe der Formel IIIc, IIId, IIIe, Cyan oder Ar bedeutet,

$-S-(O)_y-R_{10}$ $-S-(O)_y-R_{10}$ $-Si(C_6H_5)_2-CH_3$ 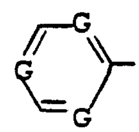

IIIc IIId IIIe

IIIc

$R^{10}$ niederes Alkyl oder Ar bedeutet.
G, das gleich oder verschieden sein kann, C oder N bedeutet, wobei jedoch mindestens ein G für N steht,
Ar Phenyl bedeutet, das durch 1 bis 5 gleiche oder verschiedene Substituenten der Reihe Chlor, Fluor, Nitro und Cyan oder zusätzlich zu den elektronenabziehenden Substituenten durch einen oder zwei lipophile Reste substituiert sein kann,
y 0, 1 oder 2 ist,
$R^9$ eine Gruppe der Formel IIIf oder IIIg bedeutet,

$CY_3$ 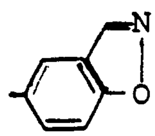

IIIf IIIg

Y gleiche oder verschiedene Substituenten der Reihe Chlor, Brom oder Jod bedeutet,
$R^2$ eine Bedeutung von $R^1$ haben kann und dann gleich oder verschieden ist, oder Alkyl mit 1 bis 4 Kohlenstofatomen, eine Gruppe der Formel IIIh

$-CH_2-Ar$ IIIh

oder auch Ar bedeutet, wenn dieses alkalisch leichter abspaltbar ist als $R^1$,
$R^5$ und $R^8$ zusammen für eine Gruppe der Formel IIIi stehen

IIIi

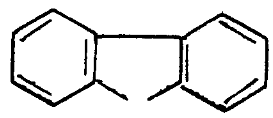

IIIi

und $R^1$ auch für Methyl steht, wenn $R^2$ ebenfalls Methyl ist,

$R^3$ und $R^4$, die gleich oder verschieden sein können, Alkyl mit 1 bis 8 Kohlenstoffatomen, Cycloalkyl mit 5 bis 12 Kohlenstoffatomen, Benzyl oder Phenyl oder zusammen mit dem Stickstoffatom, an das sie gebunden sind, einen gesättigten oder ungesättigten heterocyclischen Ring bedeuten, der gegebenenfalls weitere Heteroatome und Substituenten enthalten kann, und die erhaltene Verbindung der Formel IV

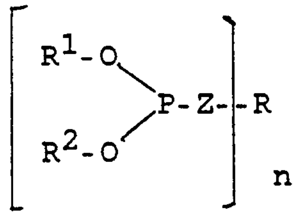

IV

in der $R^1$, $R^2$, R, Z und n die genannten Bedeutungen haben, zur Verbindung der Formel V

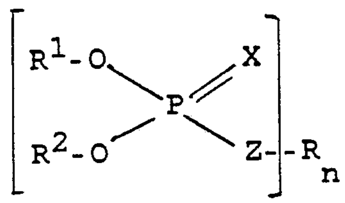

V

in der n, $R^1$, $R^2$, X, Z und R die genannten Bedeutungen haben, oxidiert.

2. Verbindungen der Formel III, in denen $R^1$ von Methyl verschieden ist.

3. Verbindungen nach Anspruch 2, in denen

$R^5$, $R^6$ und $R^7$ Wasserstoff,

$R^8$ Cyan, Phenyl oder durch Nitro, Chlor oder einen oder zwei Kohlenwasserstoffreste mit 8 bis 24 C-Atomen substituiertes Phenyl bedeutet,

$R^9$ eine Gruppe der Formel IIIf bedeutet,

$R^2$ Alkyl mit 1 bis 4 Kohlenstoffatomen bedeutet oder die Bedeutung von $R^1$ hat,

$R^3$ und $R^4$ Alkyl mit 1 bis 6 C-Atomen, Cyclohexyl, Benzyl, Phenyl und zusammen mit dem Stickstoffatom Pyrrolidyl, Piperidyl, Morpholyl, Triazolo, Benzotriazolo oder Tetrazolo bedeuten, wobei die aromatischen Reste durch Chlor, Fluor, Nitro oder Cyan substituiert sein können.

4. Verbindungen nach Anspruch 3, in denen

$R^8$ Mono- oder Dinitrophenyl, Mono-, Di- oder Trichlorphenyl, 2-Chlor-4-nitrophenyl oder zusätzlich zu den elektronenabziehenden Substituenten durch einen oder zwei Kohlenwasserstoffreste mit 12 bis 18 C-Atomen substituiertes Phenyl bedeutet,

$R^9$ Trichlormethyl ist,

$R^2$ Methyl ist und

$R^3$ und $R^4$ Alkyl mit 1 bis 4 Kohlenstoffatomen bedeuten.

**Patentansprüche** für den Vertragsstaat Österreich

1. Verfahren zur Herstellung von Verbindungen der Formel I

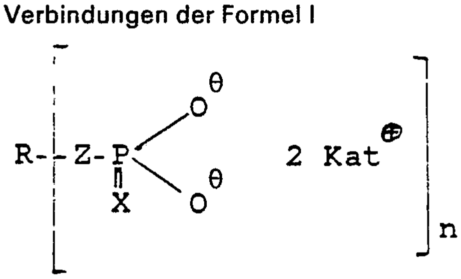

I

in der

Kat für ein Äquivalent eines Kations steht,

R ein organischer Rest ist, der keine weiteren phosphorylierbaren Wasserstoffatome trägt,
X für Sauerstoff, Schwefel oder Selen steht,
Z für Sauerstoff, Schwefel oder -NH- steht, und
n mindestens 1 bedeutet,
durch Phosphorylierung einer Verbindung der Formel II

$R\text{-}(\text{-}Z\text{-}H)_n$ II

mit mindestens n Mol eines Phosphorylierungsreagenzes, Oxidation der erhaltenen Vebrindung und Freisetzung der Verbindung der Formel I mit Basen, dadurch gekennzeichnet, daß man in Gegenwart einer schwachen Säure ein Phosphorylierungsreagens der Formel III

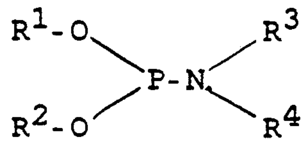
III

einsetzt,
in der $R^1$ für eine Gruppe der Formel IIIa oder IIIb steht,

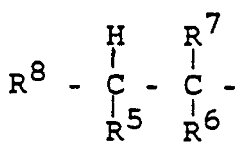

IIIa

$R^9 - CH_2 -$

IIIb

$R^5$, $R^6$ und $R^7$, die gleich oder verschieden sein können, Wasserstoff oder Methyl bedeuten,
$R^8$ eine Gruppe der Formel IIIc, IIId, IIIe, Cyan oder Ar bedeutet,

$-S-(O)_y-R_{10}$ IIIc

$-Si(C_6H_5)_2-CH_3$ IIId

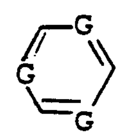
IIIe

$R^{10}$ Alkyl mit 1 bis 4 Kohlenstoffatomen oder Ar bedeutet,
G, das gleich oder verschieden sein kann, C oder N bedeutet, wobei jedoch mindestens ein G für N steht,
Ar Phenyl bedeutet, das durch 1 bis 5 gleiche oder verschiedene Substituenten der Reihe Chlor, Fluor, Nitro und Cyan oder zusätzlich zu den elektronenabziehenden Substituenten durch einen oder zwei lipophile Reste substituiert sein kann,
y 0, 1 oder 2 ist,
$R^9$ eine Gruppe der Formel IIIf oder IIIg bedeutet,

$CY_3$ IIIf

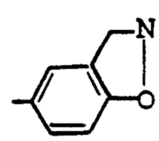
IIIg

Y gleiche oder verschiedene Substituenten der Reihe Chlor, Brom oder Jod bedeutet,
$R^2$ eine Bedeutung von $R^1$ haben kann und dann gleich oder verschieden ist, oder Alkyl mit 1 bis 4 Kohlenstoffatomen, eine Gruppe der Formel IIIh

$-CH_2-Ar$ IIIh

oder auch Ar bedeutet, wenn dieses alkalisch leichter abspaltbar ist als $R^1$,

$R^5$ und $R^8$ zusammen für eine Gruppe der Formel IIIi stehen

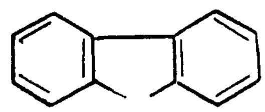

IIIi

und $R^1$ auch für Methyl steht, wenn $R^2$ ebenfalls Methyl ist,

$R^3$ und $R^4$, die gleich oder verschieden sein können, Alkyl mit 1 bis 8 Kohlenstoffatomen, Cycloalkyl mit 5 bis 12 Kohlenstoffatomen, Benzyl oder Phenyl oder zusammen mit dem Stickstoffatom, an das sie gebunden sind, einen gesättigten oder ungesättigten heterocyclischen Ring bedeuten, der gegebenenfalls weitere Heteroatome und Substituenten enthalten kann, und die erhaltene Verbindung der Formel IV

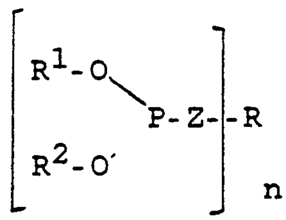

IV

in der $R^1$, $R^2$, R, Z und n die genannten Bedeutungen haben, zur Verbindung der Formel V

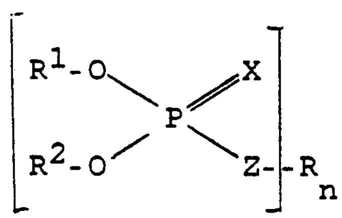

V

in der n, $R^1$, $R^2$, X, Z und R die genannten Bedeutungen haben, oxidiert.

2. Verfahren nach Anspruch 1, dadurch gekennzeichnet, daß man Verbindungen der Formel III einsetzt, in denen $R^1$ von Methyl verschieden ist.

3. Verfahren nach Anspruch 2, dadurch gekennzeichnet, daß man Verbindungen einsetzt, in denen

$R^5$, $R^6$ und $R^7$ Wasserstoff,

$R^8$ Cyan, Phenyl oder durch Nitro, Chlor oder einen oder zwei Kohlenwasserstoffreste mit 8 bis 24 C-Atomen substituiertes Phenyl bedeutet,

$R^9$ eine Gruppe der Formel IIIf bedeutet,

$R^2$ Alkyl mit 1 bis 4 Kohlenstoffatomen ist oder die Bedeutung von $R^1$ hat,

$R^3$ und $R^4$ Alkyl mit 1 bis 6 C-Atomen, Cyclohexyl, Benzyl, Phenyl und zusammen mit dem Stickstoffatom Pyrrolidyl, Piperidyl, Morpholyl, Triazolo, Benzotriazolo oder Tetrazolo bedeuten, wobei die aromatischen Reste durch Chlor, Fluor, Nitro oder Cyan substituiert sein können.

4. Verfahren nach Anspruch 3, dadurch gekennzeichnet, daß man Verbindungen einsetzt, in denen $R^8$ Mono- oder Dinitrophenyl, Mono-, Di- oder Trichlorphenyl, 2-Chlor-4-nitrophenyl oder zusätzlich zu den elektronenabziehenden Substituenten durch einen oder zwei Kohlenwasserstoffreste mit 12 bis 18 C-Atomen substituiertes Phenyl bedeutet,

$R^9$ Trichlormethyl ist,

$R^2$ Methyl ist und

$R^3$ und $R^4$ Alkyl mit 1 bis 4 Kohlenstoffatomen bedeuten.

**Claims** for the contracting states: BE, CH, DE, FR, GB, IT, LI, LU, NL, SE

1. A process for the preparation of compounds of the formula I

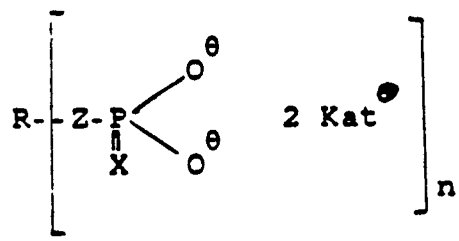

I

in which

Kat represents one molar equivalent of a cation,

R is an organic radical which carries no further phosphorylatable hydrogen atoms,

X represents oxygen, sulfur or selenium,

Z represents oxygen, sulfur or -NH- and

n denotes at least 1,

by phosphorylating a compound of the formula II

$R\text{-}(\text{-}Z\text{-}H)_n$ II

with at least n moles of a phosphorylating reagent, oxidizing the resulting compound and liberating the compound of the formula I by means of bases, characterized by employing, in the presence of a weak acid, a phosphorylating reagent of the formula III

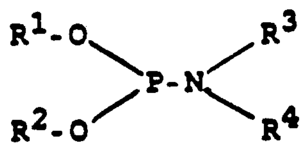

III

in which $R^1$ represents a group of the formula IIIa or IIIb

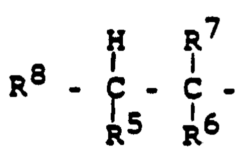

$R^9 - CH_2 -$

IIIa IIIb

$R^5$, $R^6$ and $R^7$, which can be identical or different, denote hydrogen or methyl,

$R^8$ denotes a group of the formula IIIc, IIId or IIIe or cyano or Ar

$-S-(O)_y-R_{10}$ $-Si(C_6H_5)_2-CH_3$ 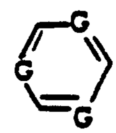

IIIc IIId IIIe

$R^{10}$ denotes lower alkyl or Ar,

G, which can be identical or different, denotes C or N, but at least one G represents N,

Ar denotes phenyl which can be substituted by 1 to 5 identical or different substituents belonging to the series chlorine, fluorine, nitro and cyano or, in addition to the electron-withdrawing substituents, can be substituted by one or two lipophilic radicals,

y is 0, 1 or 2,

$R^9$ denotes a group of the formula IIIf or IIIg

$CY_3$

IIIf

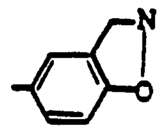

IIIg

Y denotes identical or different substituents belonging to the series chlorine, bromine or iodine,

$R^2$ can have a meaning of $R^1$ and is then identical or different, or denotes alkyl having 1 to 4 carbon atoms, a group of the formula IIIh

$-CH_2-Ar$ IIIh

or Ar, if the latter can be split off more readily than $R^1$ by alkalis,

$R^5$ and $R^8$ together represent a group of the formula IIIi

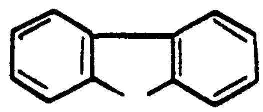

IIIi

and $R^1$ also represents methyl if $R^2$ is also methyl, and

$R^3$ and $R^4$, which can be identical or different, denote alkyl having 1 to 8 carbon atoms, cycloalkyl having 5 to 12 carbon atoms, benzyl or phenyl or, together with the nitrogen atom to which they are attached, denote a saturated or unsaturated heterocyclic ring which can, if appropriate, contain further heteroatoms and substituents,

and oxidizing the resulting compound of the formula IV

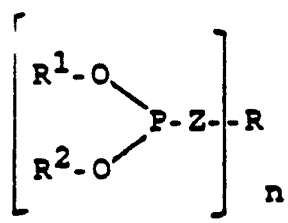

IV

in which $R^1$, $R^2$, R, Z and n have the meanings mentioned to give the compound of the formula V

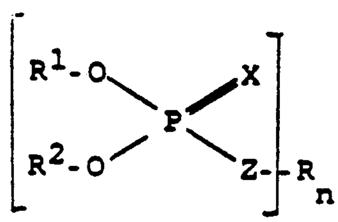

V

in which n, $R^1$, $R^2$, X, Z and R have the meanings mentioned.

2. A compound of the formula III in which $R^1$ other than methyl.

3. A compound as claimed in claim 2, in which

$R^5$, $R^6$ and $R^7$ denote hydrogen,

$R^8$ denotes cyano, phenyl or phenyl which is substituted by nitro, chlorine or one or two hydrocarbon radicals having 8 to 24 carbon atoms,

$R^9$ denotes a group of the formula IIIf,

$R^2$ denotes alkyl having 1 to 4 carbon atoms or has the meaning of $R_1$, and

$R^3$ and $R^4$ denote alkyl having 1 to 6 carbon atoms, cyclohexyl, benzyl or phenyl and, together with the nitrogen atom, denote pyrrolidyl, piperidyl, morpholyl, triazolo, benzotriazolo or tetrazolo, wherein the aromatic radicals can be substituted by chlorine, fluorine, nitro or cyano.

4. A compound as claimed in claim 3, in which

$R^8$ denotes mononitrophenyl, dinitrophenyl, monochlorophenyl, dichlorophenyl, trichlorophenyl or 2-chloro-4-nitrophenyl or phenyl which, additionally to the electron-withdrawing substituents, is substituted by one or two hydrocarbon radicals having 12 to 18 carbon atoms,

$R^9$ is trichloromethyl,

$R^2$ is methyl and

$R^3$ and $R^4$ denote alkyl having 1 to 4 carbon atoms.

**Claims** for the contracting state: Austria

1. A process for the preparation of compounds of the formula I

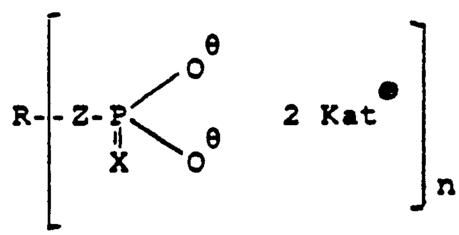

I

in which

Kat represents one molar equivalent of a cation,
R is an organic radical which carries no further phosphorylatable hydrogen atoms,
X represents oxygen, sulfur or selenium,
Z represents oxygen, sulfur or -NH- and
n denotes at least 1,

by phosphorylating a compound of the formula II

$R\text{-}(\text{-}Z\text{-}H)_n$ II

with at least n moles of a phosphorylating reagent, oxidizing the resulting compound and liberating the compound of the formula I by means of bases, characterized by employing, in the presence of a weak acid, a phosphorylating reagent of the formula III

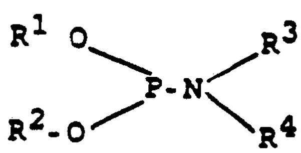

III

in which $R^1$ represents a group of the formula IIIa or IIIb

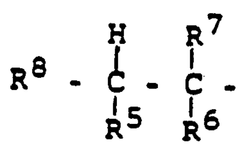

$R^9 - CH_2 -$

IIIa IIIb

$R^5$, $R^6$ and $R^7$ which can be identical or different, denote hydrogen or methyl,
$R^8$ denotes a group of the formula IIIc, IIId or IIIe or cyano or Ar

$-S-(O)_y-R_{10}$ $-Si(C_6H_5)_2-CH_3$

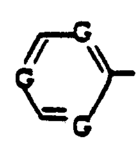

IIIc IIId IIIe

$R^{10}$ denotes alkyl having 1 to 4 carbon atoms or Ar,
G, which can be identical or different, denotes C or N, but at least one G represents N,
Ar denotes phenyl which can be substituted by 1 to 5 identical or different substituents belonging to the series chlorine, fluorine, nitro and cyano or, in addition to the electron-withdrawing substituents, can be substituted by one or two lipophilic radicals,
y is 0, 1 or 2,
$R^9$ denotes a group of the formula IIIf or IIIg

$CY_3$

IIIf

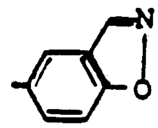

IIIg

Y denotes identical or different substituents belonging to the series chlorine, bromine or iodine,

$R^2$ can have a meaning of $R^1$ and is then identical or different, or denotes alkyl having 1 to 4 carbon atoms, a group of the formula IIIh

$-CH_2-Ar$ IIIh

or Ar, if the latter can be split off more readily than $R^1$ by alkal is,

$R^5$ and $R^8$ together represent a group of the formula IIIi

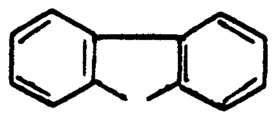

IIIi

and $R^1$ also represents methyl if $R^2$ is also methyl, and

$R^3$ and $R^4$, which can be identical or different, denote alkyl having 1 to 8 carbon atoms, cycloalkyl having 5 to 12 carbon atoms, benzyl or phenyl or, together with the nitrogen atom to which they are attached, denote a saturated or unsaturated heterocyclic ring which can, if appropriate, contain further heteroatoms and substituents,

and oxidizing the resulting compound of the formula IV

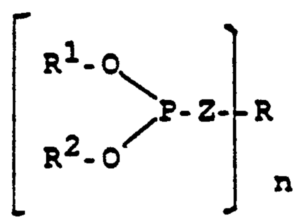

IV

in which $R^1$, $R^2$, R, Z and n have the meanings mentioned to give the compound of the formula V

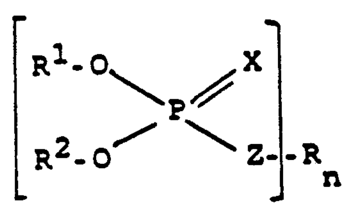

V

in which n, $R^1$, $R^2$, X, Z and R have the meanings mentioned.

2. The process as claimed in claim 1, characterized in that compounds of the formula III in which $R^1$ other than methyl are employed.

3. The process as claimed in claim 2, characterized in that compounds are employed in which

$R^5$, $R^6$ and $R^7$ denote hydrogen,

$R^8$ denotes cyano, phenyl or phenyl which is substituted by nitro, chlorine or one or two hydrocarbon radicals having 8 to 24 carbon atoms,

$R^9$ denotes a group of the formula IIIf,

$R^2$ is alkyl having 1 to 4 carbon atoms or has the meaning of $R^1$, and

$R^3$ and $R^4$ denote alkyl having 1 to 6 carbon atoms, cyclohexyl, benzyl or phenyl and, together with the nitrogen atom, denote pyrrolidyl, piperidyl, morpholyl, triazolo, benzotriazolo or tetrazolo, wherein

the aromatic radicals can be substituted by chlorine, fluorine, nitro or cyano.

4. The process as claimed in claim 3, characterized in that compounds in which

$R^8$ denotes mononitrophenyl, dinitrophenyl, monochlorophenyl, dichlorophenyl, trichlorophenyl or 2-Chloro-4-nitrophenyl or phenyl which, additionally to the electron-withdrawing substituents, is substituted by one or two hydrocarbon radicals having 12 to 18 carbon atoms,

$R^9$ is trichloromethyl,

$R^2$ is methyl and

$R^3$ and $R^4$ denote alkyl having 1 to 4 carbon atoms, are employed.

**Revendications** pour les Etats Contractants: BE, CH, DE, FR, GB, IT, LI, LU, NL, SE

1. Procédé pour la préparation de composés de formule I

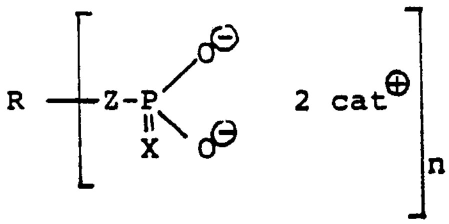

I

dans laquelle
cat représente un équivalent d'un cation,
R est un radical organique qui ne porte pas d'autres atomes d'hydrogène phosphorylables,
X représente un atome d'oxygène, de soufre ou de sélénium,
Z représente un atome d'oxygène, de soufre ou -NH-,
n vaut au moins 1,
par phosphorylation d'un composé de formule II

$$R\text{-}(\text{-}Z\text{-}H)_n \qquad \text{II}$$

avec au moins n moles d'un réactif de phosphorylation, oxydation du composé obtenu et libération, à l'aide de bases, du composé de formule I, caractérisé en ce que l'on fait réagir, en présence d'un acide faible, un réactif de phosphorylation de formule III

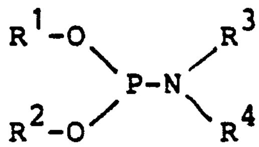

III

dans laquelle
$R^1$ représente un groupe de formule IIIa ou IIIb

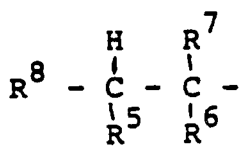

IIIa

$$R^9 - CH_2-$$

IIIb

$R^5$, $R^6$ et $R^7$, qui peuvent être identiques ou différents, représentent un atome d'hydrogène ou le groupe méthyle;
$R^8$ représente un groupe de formule IIIc, IIId, IIIe, cyano ou Ar,

$$-S-(O)_y-R_{10}$$

IIIc

$$-Si(C_6H_5)_2-CH_3$$

IIId

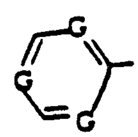

IIIe

$R^{10}$ représente un groupe alkyle inférieur ou Ar, et les atomes G, qui peuvent être identiques ou différents, représentent C ou N, mais au moins un G représente N, Ar représente un radical phényle qui peut être substitué par 1 à 5 substituants identiques ou différents, choisis parmi des atomes de chlore ou de fluor, ou des groupes nitro et cyano, ou en plus des substituants électro-attracteurs, par un ou deux restes lipophiles,
y vaut 0, 1 ou 2,
$R^9$ représente un groupe de formule IIIf ou IIIg,

$CY_3$ 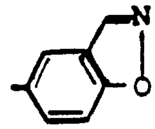

IIIf IIIg

Y représentant des substituants identiques ou différents, choisis parmi des atomes de chlore, brome ou iode, $R^2$ peut avoir une des significations de $R^1$ et être alors identique à ou différent de celui-ci, ou représenter un groupe alkyle ayant de 1 à 4 atomes de carbone, un groupe de formule IIIh

$-CH_2-Ar$ IIIh

ou bien Ar, lorsque celui-ci est plus aisément séparable par des alcalis que $R^1$,

$R^5$ et $R^8$ peuvent représenter ensemble un groupe de formule IIIi

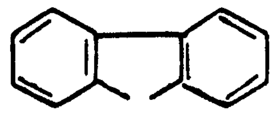

IIIi

et

$R^1$ peut aussi représenter un groupe méthyle, lorsque $R^2$ représente également un groupe méthyle.

$R^3$ et $R^4$ qui peuvent être identiques ou différents, représentent des groupes alkyle ayant de 1 à 8 atomes de carbone, cycloalkyle ayant de 5 à 12 atomes de carbone, le radical benzyle ou phényle ou forment ensemble, conjointement avec l'atome d'azote auquel ils sont liés, un cycle hétérocyclique saturé ou non saturé, qui peut éventuellement contenir d'autres hétéroatomes et substituants,
et on oxyde le composé de formule IV obtenu

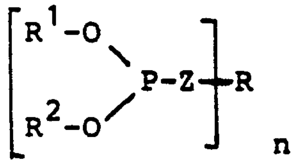

IV

dans laquelle $R^1$, $R^2$, Z et n ont les significations indiquées, pour aboutir au composé de formule V

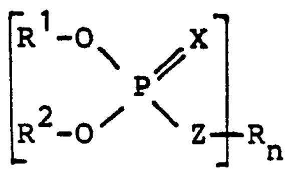

V

dans laquelle n, $R^1$, $R^2$, X, Z et R ont les significations indiquées.

2. Composés de formule III, dans lesquels $R^1$ est différent d'un groupe méthyle.

3. Composés selon la revendication 2, dans lesquels

$R^5$, $R^6$ et $R^7$ représentent un atome d'hydrogène,

$R^8$ représente un radical cyano, phényle ou phényle substitué par un atome de chlore ou par un groupe nitro ou par un ou deux restes hydrocarbonés ayant de 8 à 24 atomes de carbone,

$R^9$ représente un groupe de formule IIIf,

$R^2$ représente un groupe alkyle ayant de 1 à 4 atomes de carbone, ou a la signification de $R^1$,

$R^3$ et $R^4$ représentent un groupe alkyle ayant de 1 à 6 atomes de carbone, un radical cyclohexyle, benzyle, phényle, ou forment ensemble, conjointement avec l'atome d'azote, un radical pyrrolidyle, pipéridyle, morpholyle, triazolo, benzotriazolo ou tétrazolo, les radicaux aromatiques pouvant être substitués par des atomes de chlore ou de fluor ou par des groupes nitro ou cyano.

4. Composés selon la revendication 3, dans lesquels

$R^8$ représente un groupe mono- ou dinitrophényle, mono-, di- ou trichlorophényle, 2-chloro-4-nitrophényle, ou un radical phényle substitué, en plus des substituants attirant les électrons, par un ou deux restes hydrocarbones ayant de 12 à 18 atomes de carbone,

$R^9$ est le radical trichlorométhyle,

$R^2$ est le groupe méthyle, et

$R^3$ et $R^4$ représentent un groupe alkyle ayant de 1 à 4 atomes de carbone.

**Revendications** pour l'Etat Contractant: AT

1. Procédé pour la préparation de composés de formule I

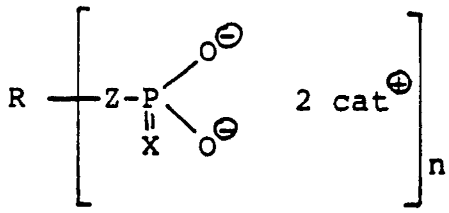

I

dans laquelle

cat représente un équivalent d'un cation,
R est un radical organique qui ne porte pas d'autres atomes d'hydrogène phosphorylables,
X représente un atome d'oxygène, de soufre ou de sélénium,
Z représente un atome d'oxygène, de soufre ou -NH-,
n vaut au moins 1,
par phosphorylation d'un compose de formule II

$R\text{-}(\text{-}Z\text{-}H)_n$ II

avec au moins n moles d'un réactif de phosphorylation, oxydation du composé obtenu et libération, à l'aide de bases, du composé de formule caractérisé en ce que l'on fait réagir, en présence d'un acide faible, un réactif de phosphorylation de formule III

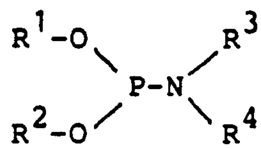

III

dans laquelle
$R^1$ représente un groupe de formule IIIa ou IIIb

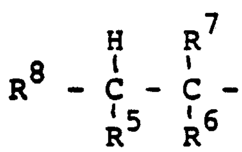

IIIa

$R^9 - CH_2-$

IIIb

$R^5$, $R^6$ et $R^7$ qui peuvent être identiques ou différents, représentent un atome d'hydrogène ou le groupe méthyle;
$R^8$ représente un groupe de formule IIIc, IIId, IIIe, cyano ou Ar,

$-S-(O)_y-R_{10}$ IIIc

$-Si(C_6H_5)_2-CH_3$ IIId

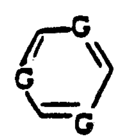

IIIe

$R^{10}$ représente un groupe alkyle ayant de 1 à 4 atomes de carbone ou Ar, et
les atomes G, qui peuvent être identiques ou différents, représentent C ou N, mais au moins un G représente N, Ar représente un radical phényle qui peut être substitué par 1 à 5 substituants identiques ou différents, choisis parmi des atomes de chlore ou de fluor, ou des groupes nitro et cyano, ou en plus des substituants électro-attracteurs, par un ou deux restes lipophiles,
y vaut 0, 1 ou 2,
$R^9$ représente un groupe de formule IIIf ou IIIg,

$CY_3$ 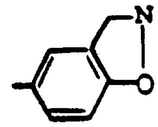

IIIf IIIg

Y représentant des substituants identiques ou différents, choisis parmi des atomes de chlore, brome ou iode, $R^2$ peut avoir une des significations de $R^1$ et être alors identique à ou différent de celui-ci, ou représenter un groupe alkyle ayant de 1 à 4 atomes de carbone, un groupe de formule IIIh

$-CH_2-Ar$ IIIh

ou bien Ar, lorsque celui-ci est plus aisément séparable par des alcalis que $R^1$,

$R^5$ et $R^8$ peuvent représenter ensemble un groupe de formule IIIi

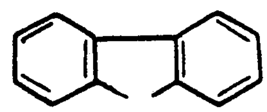

IIIi

et

$R^1$ peut aussi représenter un groupe méthyle, lorsque $R^2$ représente également un groupe méthyle.

$R^3$ et $R^4$, qui peuvent être identiques ou différents, représentent des groupes alkyle ayant de 1 à 8 atomes de carbone, cycloalkyle ayant de 5 à 12 atomes de carbone, le radical benzyle ou phényle, ou forment ensemble, conjointement avec l'atome d'azote auquel ils sont liés, un cycle hétérocyclique saturé ou non saturé, qui peut éventuellement contenir d'autres hétéroatomes et substituants,

et on oxyde le composé de formule IV obtenu

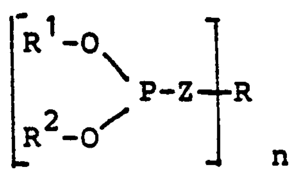

IV

dans laquelle $R^1$, $R^2$, Z et n ont les significations indiquées, pour aboutir au composé de formule V

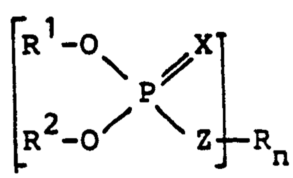

V

dans laquelle n, $R^1$, $R^2$, X, Z et R ont les significations indiquées.

2. Procédé selon la revendication 1, caractérisé en ce que l'on utilise des composés de formule III, dans lesquels $R^1$ est différent d'un groupe méthyle.

3. Procédé selon la revendication 2, caractérisé en ce que l'on utilise des composés dans lesquels

$R^5$, $R^6$ et $R^7$ représentent un atome d'hydrogène,

$R^8$ représente un radical cyano, phényle ou phényle substitué par un atome de chlore ou par un groupe nitro ou par un ou deux restes hydrocarbonés ayant de 8 à 24 atomes de carbone,

$R^9$ représente un groupe de formule IIIf,

$R^2$ représente un groupe alkyle ayant de 1 à 4 atomes de carbone, ou a la signification de $R^1$,

$R^3$ et $R^4$ représentent un groupe alkyle ayant de 1 à 6 atomes de carbone, un radical cyclohexyle, benzyle, phényle, ou forment ensemble, conjointement avec l'atome d'azote, un radical pyrrolidyle, pipéridyle, morpholyle, triazolo, benzotriazolo ou tétrazolo, les radicaux aromatiques pouvant être substitués par des atomes de chlore ou de fluor ou par des groupes nitro ou cyano.

4. Procédé selon la revendication 3, caractérisé en ce que l'on utilise des composés dans lesquels $R^8$ représente un groupe mono- ou dinitrophényle, mono-, di- ou trichlorophényle, 2-chloro-4-nitrophényle, ou un radical phényle substitué, en plus des substituants attirant les électrons, par un ou deux restes hydrocarbonés ayant de 12 à 18 atomes de carbone,

$R^9$ est le radical trichlorométhyle,

$R^2$ est le groupe méthyle, et

$R^3$ et $R^4$ représentent un groupe alkyle ayant de 1 à 4 atomes de carbone.